(19) Europäisches Patentamt
European Patent Office
Office européen des brevets

(11) EP 3 015 451 B1

(12) EUROPEAN PATENT SPECIFICATION

(45) Date of publication and mention of the grant of the patent:
**17.01.2018 Bulletin 2018/03**

(21) Application number: **14817123.4**

(22) Date of filing: **03.06.2014**

(51) Int Cl.:
*C07C 205/12* (2006.01) *C07C 205/58* (2006.01)
*C07B 61/00* (2006.01) *C07C 201/10* (2006.01)

(86) International application number:
**PCT/JP2014/064780**

(87) International publication number:
**WO 2014/208296 (31.12.2014 Gazette 2014/53)**

(54) **METHOD FOR PRODUCING NITROBENZENE COMPOUND**

VERFAHREN ZUR HERSTELLUNG EINER NITROBENZOLVERBINDUNG

PROCÉDÉ DE PRODUCTION D' UN COMPOSÉ DE NITROBENZÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2013 JP 2013133148**

(43) Date of publication of application:
**04.05.2016 Bulletin 2016/18**

(73) Proprietor: **Kumiai Chemical Industry Co., Ltd.**
**Taito-ku**
**Tokyo 110-0008 (JP)**

(72) Inventors:
- **IKUMI, Akiko**
  **Fuji-shi**
  **Shizuoka 421-3306 (JP)**
- **NAKAMURA, Takayuki**
  **Fuji-shi**
  **Shizuoka 421-3306 (JP)**

(74) Representative: **Beckmann, Claus et al**
**Kraus & Weisert**
**Patentanwälte PartGmbB**
**Thomas-Wimmer-Ring 15**
**80539 München (DE)**

(56) References cited:
**EP-A1- 1 247 810** **WO-A1-2006/018443**
**DE-C- 638 486** **JP-B2- 2 606 291**
**US-A- 4 032 639**

- **FENG RONG-XIU ET AL: "Synthesis of 3-Chloro-2- Nitrotoluene", TRANSACTIONS OF TIANJIN UNIVERSITY, EDITORIAL BOARD OF TRANSACTIONS OF TIANJIN UNIVERSITY, TIANJIN, CN, vol. 8, no. 1, 1 March 2002 (2002-03-01), pages 40-42, XP008182596, ISSN: 1006-4982**
- **FENG RONG-XIU ET AL.: 'Synthesis of 3-Chloro-2- Nitrotoluene' TRANSACTIONS OF TIANJIN UNIVERSITY vol. 8, no. 1, March 2002, pages 40 - 42, XP008182596**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 015 451 B1

## Description

### Technical Field

The present invention relates to a method for producing a nitrobenzene compound represented by the general formula (1) :

[Chemical Formula 1]

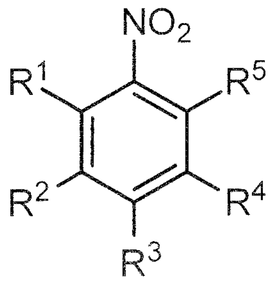

(1)

wherein $R^1$ represents a halogen atom, $R^2$, $R^3$, and $R^4$ are the same or different from one another and each represent a hydrogen atom, a halogen atom, or an alkyl group, and $R^5$ represents a halogen atom or an alkoxycarbonyl group.

### Background Art

A nitrobenzene compound of the general formula (1) is useful as an intermediate used to manufacture various beneficial organic compounds. Particularly, 2,6-dichloronitrobenzene (see Patent Documents 1 and 2) and 2-chloro-6-alkoxycarbonyl nitrobenzenes (see Patent Documents 3 and 4) are known as intermediates used to manufacture medicines and agricultural chemicals, and the like.

As a method for producing a nitrobenzene compound of the general formula (1), a producing method is known in which an aniline compound is diazotized and then nitrated (see Patent Document 5 and Non Patent Document 1). However, this method has some problems. One of the problems is that economic efficiency is low due to the use of a large amount of water as a solvent. Another problem is that a dinitro compound is formed as a by-product. Here, the dinitro compound refers to a dinitrobenzene compound. That is, the dinitro compound refers to a compound having two nitro groups on the benzene ring. Such dinitro compounds are generally known to be very dangerous. From an industrial viewpoint, formation of the dinitro compound is not preferred even if its amount is small. However, as shown in Comparative Example 1, when this method was used, the dinitro compound was formed in an amount as large as 3% or more. The formation of the dinitro compound as a by-product in an amount as large as 3% is a problem that should be improved. That is, a method for reducing the amount of the dinitro compound formed as a by-product is desired.

Meanwhile, as another method for producing a nitrobenzene compound of the general formula (1), a method is known in which an aniline compound is oxidized by hydrogen peroxide (see Patent Document 6). This method uses hydrogen peroxide that should be carefully handled to keep the safety of industrial processes. The method described in Patent Document 6 is superior to conventional art known before Patent Document 6, but still has a room for improvement in the use of hydrogen peroxide.

### Citation List

### Patent Document

Patent Document 1: JP 2005-533756 A
Patent Document 2: JP 2008-537953 A
Patent Document 3: WO 2005/081960 A2
Patent Document 4: US 5084086 A
Patent Document 5: JP 2606291 B2
Patent Document 6: WO 2013/005425 A1

Non Patent Document

Non Patent Document 1: Transactions of Tianj in University, 2002, Vol. 8, No. 1, pp. 40-41

Summary of the Invention

Problems to be Solved by the Invention

It is an object of the present invention to provide a method for producing a nitrobenzene compound of the general formula (1), the method being capable of improving economic efficiency as compared to conventional art. More specifically, for example, it is an object of the present invention to provide a method for producing a nitrobenzene compound of the general formula (1) using a reduced amount of water.

It is another object of the present invention to provide a method for producing a nitrobenzene compound of the general formula (1), the method providing improved safety. More specifically, for example, it is another object of the present invention to provide a method for producing a nitrobenzene compound of the general formula (1), the method being capable of reducing the amount of a dinitro compound formed as a by-product.

It is yet another obj ect of the present invention to provide a method for producing a nitrobenzene compound of the general formula (1) without using hydrogen peroxide.

That is, it is an object of the present invention to provide a method for producing a nitrobenzene compound of the general formula (1), the method being economically favorable and suitable for industrialization.

Means for Solving the Problems

In view of the above-mentioned circumstances, the present inventors have intensively studied a method for producing a nitrobenzene compound of the general formula (1). As a result, the present inventors have unexpectedly found that a safe industrial producing method can be provided which is capable of suppressing the formation of a polynitro compound, such as a dinitro compound, as a by-product by adjusting the total amount of water used in a reaction system. That is, the present inventors have found that the above problem can be solved by providing a method for producing a nitrobenzene compound represented by the general formula (1):

[Chemical Formula 2]

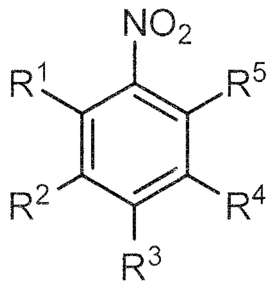

(1)

wherein $R^1$ represents a halogen atom, $R^2$, $R^3$, and $R^4$ are the same or different from one another and each represent a hydrogen atom, a halogen atom, or an alkyl group, and $R^5$ represents a halogen atom or an alkoxycarbonyl group, the method including: reacting an aniline compound represented by the general formula (2) :

[Chemical Formula 3]

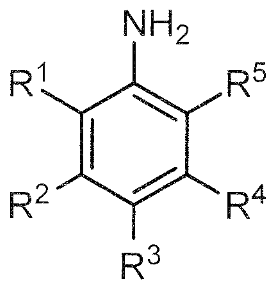

(2)

wherein $R^1$, $R^2$, $R^3$, $R^4$, and $R^5$ are as defined above, in the presence of a metal nitrite and an acid; and further reacting a resulting product or a reaction mixture containing the resulting product in the presence of a metal nitrite and a copper compound; and a total amount of water used in the reaction is 1. 2 to 2.2 L per 1 mol of the compound represented by the general formula (2). The present inventors have completed the present invention based on this finding.

More specifically, the present invention provides the following.

[1] A method for producing a nitrobenzene compound represented by the general formula (1):

[Chemical Formula 4]

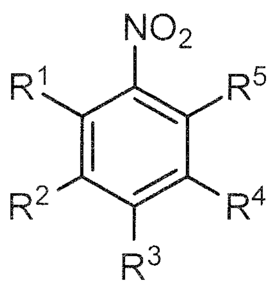

(1)

wherein $R^1$ represents a halogen atom, $R^2$, $R^3$, and $R^4$ are the same or different from one another and each represent a hydrogen atom, a halogen atom, or an alkyl group, $R^5$ represents a halogen atom or an alkoxycarbonyl group, the method including the processes of: (i) reacting an aniline compound represented by the general formula (2):

[Chemical Formula 5]

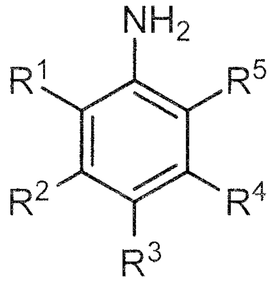

(2)

wherein $R^1$, $R^2$, $R^3$, $R^4$, and $R^5$ are as defined above, in the presence of a metal nitrite and an acid; and

(ii) reacting a product of the process (i) in the presence of a metal nitrite and a copper compound, wherein a total amount of water used in the process (ii) is 1.2 to 2.2 L per 1 mol of the compound of the general formula (2).

[2] The method according to the above [1], wherein a total amount of water used in the process (ii) is 1.2 to 1.6 L per 1 mol of the compound of the general formula (2).
[3] The method according to the above [1] or [2], wherein the metal nitrite used in the process (i) or the process (ii) is an alkali metal nitrite or an alkaline-earth metal nitrite.
[4] The method according to the above [1] or [2], wherein an amount of the metal nitrite used in the process (i) is 1.0 to 10.0 mol per 1 mol of the compound of the general formula (2).
[5] The method according to any one of the above [1] to [4], wherein the acid used in the process (i) is an acid selected from the group consisting of a hydrohalic acid, sulfuric acid, and phosphoric acid.
[6] The method according to any one of the above [1] to [5], wherein the copper compound used in the process (ii) is copper oxide, a copper(I) salt, or a copper(II) salt.
[7] The method according to any one of the above [1] to [6], wherein $R^2$, $R^3$, and $R^4$ are hydrogen atoms.
[8] The method according to any one of the above [1] to [7], wherein $R^1$ is a halogen atom and $R^5$ is a $C_1$ to $C_4$ alkoxycarbonyl group.
[9] The method according to any one of the above [1] to [7], wherein $R^1$ is a chlorine atom and $R^5$ is a $C_1$ to $C_4$ alkoxycarbonyl group.
[10] The method according to any one of the above [1] to [7], wherein $R^1$ is a chlorine atom and $R^5$ is methoxycarbonyl group.

[11] The method according to any one of the above [1] to [7], wherein $R^1$ is a halogen atom and $R^5$ is a halogen atom.
[12] The method according to any one of the above [1] to [7], wherein $R^1$ is a chlorine atom and $R^5$ is a chlorine atom.
[13] The method according to any one of the above [1] to [6], wherein in the process (ii), the product of the process (i) is reacted with 2.0 to 4.0 mol of metal nitrite relative to 1 mol of the compound of the general formula (2).
[14] The method according to the above [13], wherein in the process (ii), the product of the process (i) is reacted with 2.0 to 4.0 mol of metal nitrite relative to 1 mol of the compound of the general formula (2), and the total amount of water used in the process (ii) is 1.2 to 1.6 L per 1 mol of the compound of the general formula (2).
[15] The method according to the above [13] or [14], wherein $R^2$, $R^3$, and $R^9$ are hydrogen atoms.
[16] The method according to any one of the above [13] to [15], wherein $R^1$ is a halogen atom and $R^5$ is a $C_1$ to $C_4$ alkoxycarbonyl group.
[17] The method according to any one of the above [13] to [15], wherein $R^1$ is a chlorine atom and $R^5$ is a $C_1$ to $C_4$ alkoxycarbonyl group.
[18] The method according to any one of the above [13] to [15], wherein $R^1$ is a chlorine atom and $R^5$ is a methoxycarbonyl group.
[19] The method according to any one of the above [13] to [15], wherein $R^1$ is a halogen atom and $R^5$ is a halogen atom.
[20] The method according to any one of the above [13] to [15], wherein $R^1$ is a chlorine atom and $R^5$ is a chlorine atom.

Advantageous Effects of the Invention

According to the present invention, it is possible to provide a novel method for producing a nitrobenzene compound of the general formula (1).

According to the present invention, the amount of water used to manufacture a nitrobenzene compound of the general formula (1) can be reduced as compared to conventional art. Therefore, the method according to the present invention can improve economic efficiency as compared to conventional art.

On the other hand, as shown in Comparative Example 1, it was found that when the amount of water used was reduced too much, a large amount of a dinitro compound was formed as a by-product (see Comparative Example 1).

However, according to the present invention, the amount of a dangerous dinitro compound formed as a by-product can be reduced by appropriately reducing the amount of water used (see Example 1 and Comparative Example 1). Therefore, the method according to the present invention provides improved safety. In other words, the method according to the present invention can be safely applied to large-scale producing such as pilot plant-scale production or industrial-scale production. Further, according to the present invention, it is possible to manufacture a high-purity nitrobenzene compound of the general formula (1).

That is, according to the present invention, it is possible to provide a method for producing a nitrobenzene compound of the general formula (1), which includes appropriately adjusting the amount of water used and is therefore economically favorable and suitable for industrialization.

Further, according to the present invention, a nitrobenzene compound of the general formula (1) can be manufactured without using hydrogen peroxide. Also from such a viewpoint, the method according to the present invention is suitable for industrialization.

Further, in the present invention, it has also been found that the amount of a metal nitrite used to manufacture a nitrobenzene compound of the general formula (1) can be significantly reduced as compared to conventional art. In other words, according to the present invention, it is possible to provide a method for producing a nitrobenzene compound of the general formula (1) without using a large excess of a metal nitrite as compared to conventional art.

In addition, in the present invention, a base can be added to the reaction system. For example, it can be added after the completion of the reaction of the process (i) or before the start of the reaction of the process (ii). In other words, in the present invention, the pH of a reaction system of the process (ii) can be adjusted in the process (ii) by adding a base. In the present invention, a sufficient and acceptable yield can be achieved without using a base (see Example 3). However, a base may be added to achieve a higher yield, as desired (see Example 5).

The method according to the present invention provides a high yield. In addition, the method according to the present invention can be implemented by simple operation under mild conditions without using a specialized reaction apparatus. Therefore, the method according to the present invention can be efficiently implemented on an industrial scale.

Further, the present invention is considered to produce a little amount of waste and to have no problem with the properties of the waste.

Therefore, the present invention is economical and friendly to the environment and has a great deal of potential in industry.

Description of Embodiments

Hereinafter, the present invention will be described in detail.

Terms and symbols used in this specification will be described below.

As a halogen atom, a fluorine atom, a chlorine atom, a bromine atom, or an iodine atom, preferably a fluorine atom or a chlorine atom, more preferably a chlorine atom is exemplified.

The term "$C_a$ to $C_b$" means that the number of carbon atoms is from a to b. For example, the term "$C_1$ to $C_4$" in the term "$C_1$ to $C_4$ alkyl group" means that the number of carbon atoms of the alkyl group is 1 to 4.

An alkyl group is, for example, a $C_1$ to $C_4$ alkyl group. As a specific example of the $C_1$ to $C_4$ alkyl group, methyl, ethyl, propyl, isopropyl, butyl, sec-butyl, isobutyl, or tert-butyl, preferably methyl, ethyl, propyl, or isopropyl, more preferably methyl or ethyl, even more preferably methyl is exemplified.

An alkoxycarbonyl group is, for example, a $C_1$ to $C_4$ alkoxycarbonyl group. As a specific example of the $C_1$ to $C_4$ alkoxycarbonyl group, methoxycarbonyl, ethoxycarbonyl, propoxycarbonyl, isopropoxycarbonyl, butoxycarbonyl, sec-butoxycarbonyl, isobutoxycarbonyl, or tert-butoxycarbonyl, preferably methoxycarbonyl, ethoxycarbonyl, propoxycarbonyl, isopropoxycarbonyl, more preferably methoxycarbonyl or ethoxycarbonyl, even more preferably methoxycarbonyl is exemplified.

(Process (i))

First, a process (i) will be described.

The process (i) is a process in which an aniline compound represented by the general formula (2):

[Chemical Formula 6]

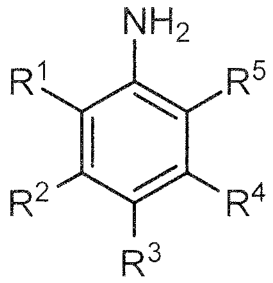

(2)

wherein $R^1$ represents a halogen atom, $R^2$, $R^3$, and $R^4$ are the same or different from one another and each represent a hydrogen atom, a halogen atom, or an alkyl group, and $R^5$ represents a halogen atom or an alkoxycarbonyl group, is reacted in the presence of a metal nitrite and an acid.

(Raw Material: Aniline Compound of General Formula (2))

A method according to the present invention uses, as a raw material, the aniline compound of the above general formula (2). The aniline compound of the general formula (2) is a known compound or a compound that can be manufactured from a known compound by a known method. Specific examples of the aniline compound of the general formula (2) include, but are not limited to, 2,6-dichloroaniline, 2,6-dibromoaniline, 2,6-difluoroaniline, 2-chloro-6-fluoroaniline, 2-bromo-6-chloroaniline, 2-chloro-6-iodoaniline, methyl 2-animo-3-chlorobenzoate, ethyl 2-amino-3-chlorobenzoate, propyl 2-amino-3-chlorobenzoate, isopropyl 2-amino-3-chlorobenzoate, butyl 2-amino-3-chlorobenzoate, isobutyl 2-amino-3-chlorobenzoate, sec-butyl 2-amino-3-chlorobenzoate, tert-butyl 2-amino-3-chlorobenzoate, methyl 2-amino-3-fluorobenzoate, ethyl 2-amino-3-fluorobenzoate, propyl 2-amino-3-fluorobenzoate, isopropyl 2-amino-3-fluorobenzoate, butyl 2-amino-3-fluorobenzoate, isobutyl 2-amino-3-fluorobenzoate, sec-butyl 2-amino-3-fluorobenzoate, tert-butyl 2-amino-3-fluorobenzoate, methyl 2-amino-3-bromobenzoate, ethyl 2-amino-3-bromobenzoate, propyl 2-amino-3-bromobenzoate, isopropyl 2-amino-3-bromobenzoate, methyl 2-amino-3-iodobenzoate, and ethyl 2-amino-3-iodobenzoate. In addition, the aniline compound of the general formula (2) may be a salt with an acid such as hydrochloric acid or sulfuric acid.

(Metal Nitrite Used in Process (i))

Examples of the metal nitrite that can be used in the process (i) include, but are not limited to, an alkali metal nitrite (e.g., lithium nitrite, sodium nitrite, or potassium nitrite) and an alkaline-earth metal nitrite (e.g., magnesium nitrite, calciumnitrite, or barium nitrite) . From the viewpoint of price, availability, and reactivity, the metal nitrite is preferably an alkali metal nitrite, more preferably sodium nitrite or potassium nitrite, even more preferably sodium nitrite.

The metal nitrite may be in any form as long as the reaction proceeds. As a form of metal nitrite, a solid consisted

of solely metal nitrite(s), an aqueous solution of metal nitrite(s) of any concentration, or a solution of metal nitrite(s) solved by a solvent other than water is exemplified. Further, the alkali metal nitrites may be used singly or in combination of two or more species of them at any ratio.

(Amount of Metal Nitrite Used in Process (i))

The amount of the metal nitrite used is not particularly limited as long as the reaction proceeds. From the viewpoint of yield, suppression of by-product formation, and economic efficiency, the amount of the metal nitrite used is, for example, usually in the range of 1.0 to 10.0 mol, preferably 1.0 to 5.0 mol, more preferably 1.0 to 3.0 mol, even more preferably 1.0 to 2.0 mol, particularly preferably 1.0 to 1.2 mol per 1 mol of the aniline compound of the general formula (2).

(Acid Used in Process (i))

Examples of the acid that can be used in the process (i) include, but are not limited to, a hydrohalic acid (e.g., hydrochloric acid, hydrobromic acid, or hydrofluoric acid), sulfuric acid, and phosphoric acid. From the viewpoint of price, handleability, yield, and suppression of by-product formation, the acid is preferably hydrochloric acid, hydrobromic acid, or sulfuric acid, more preferably hydrochloric acid or sulfuric acid, even more preferably hydrochloric acid.

The acid may be in any form as long as the reaction proceeds. As a form of the acid, liquid or gas consisting of solely acid(s), an aqueous solution of acid (s) of any concentration, or a solution of acid(s) solved by a solvent other than water is exemplified. Further, the acids may be used singly or in combination of two or more species of them at any ratio.

(Amount of Acid Used in Process (i))

The amount of the acid used is not particularly limited as long as the reaction proceeds. From the viewpoint of yield, suppression of by-product formation, and economic efficiency, the amount of the acid used is, for example, usually in the range of 1 to 10 mol, preferably 1 to 5 mol, more preferably 1 to 3 mol per 1 mol of the aniline compound of the general formula (2).

(Solvent Used in Process (i))

From the viewpoint of allowing the reaction to smoothly proceed, the reaction of the process (i) is preferably performed in the presence of solvents. The solvent used in the process (i) is not particularly limited as long as the reaction of the process (i) proceeds and the reaction of a process (ii) is not adversely affected. From the viewpoint of price and handleability, the solvent used in the process (i) is particularly preferably water.

However, solvents other than water, which will be described later, are not excluded as long as a desired reaction proceeds. For example, water and a solvent other than water may be used as long as a desired reaction proceeds. Examples of the solvent, other than water, that can be used in the process (i) include, but are not limited to: alcohols (e.g., methanol, ethanol, 2-propanol, and butanol); ethers (e.g., tetrahydrofuran (THF), 1,4-dioxane, diisopropyl ether, dibutyl ether, cyclopentyl methyl ether (CPME), methyl-tert-butyl ether, 1,2-dimethoxyethane (DME), diglyme, and triglyme); nitriles (e.g., acetonitrile); amides (e.g., N,N-dimethylformamide (DMF), N,N-dimethylacetamide (DMAC), and N-methylpyrrolidone (NMP)); alkyl ureas (e.g., N,N'-dimethylimidazolidinone (DMI)); sulfoxides (e.g., dimethylsulfoxide (DMSO)); sulfones (e.g., sulfolane); ketones (e.g., acetone and isobutyl methyl ketone (MIBK)); carboxylates (e.g., ethyl acetate and butyl acetate); carboxylic acids (e.g., acetic acid); aromatic hydrocarbon derivatives (e.g., benzene, toluene, xylene, chlorobenzene, dichlorobenzene, trichlorobenzene, and nitrobenzene); and halogenated aliphatic hydrocarbons (e.g., dichloromethane).

From the viewpoint of price, handleability, reactivity, and yield, examples of the solvent, other than water, that can be used in the process (i) preferably include alcohols, ethers, nitriles, amides, sulfoxides, ketones, aromatic hydrocarbon derivatives, and halogenated aliphatic hydrocarbons, more preferably include ketones. Specific examples of the solvent, other than water, that can be used in the process (i) preferably include methanol, ethanol, tetrahydrofuran (THF), acetonitrile, N,N-dimethylformamide (DMF), N,N-dimethylacetamide (DMAC), N-methylpyrrolidone (NMP), dimethylsulfoxide (DMSO), isobutyl methyl ketone (MIBK), toluene, xylene, chlorobenzene, dichlorobenzene, trichlorobenzene, and dichloromethane, more preferably include isobutyl methyl ketone (MIBK).

(Amount of Solvent Used in Process (i))

The amount of the solvent used in the process (i) is not particularly limited as long as a reaction system can

be sufficiently stirred. From the viewpoint of reactivity, suppression of by-product formation, and economic efficiency, the total amount of water used in the process (i) is, for example, usually in the range of 0.1 to 1.2 L (liters), preferably 0.3 to 1.0 L, more preferably 0.4 to 0.9 L per 1 mol of the aniline compound of the general formula (2). Here, the total amount of water used in the process (i) refers to the amount of all water present in the reaction system at the time when the reaction of the process (i) is finished. Therefore, when the acid or the metal nitrite added is in the form of an aqueous solution, not only the amount of water added as a solvent but also the amount of water in the aqueous solution is added. For example, the total amount of water used in the process (i) includes the amount of water in an aqueous solution of the acid used in the process (i) and the amount of water in an aqueous solution of the metal nitrite used in the process (i). Further, from the same point of view, the amount of the solvent other than water is, for example, usually in the range of 0 (zero) to 5 L (liters), preferably 0 to 1 L per 1 mol of the aniline compound of the general formula (2). It is to be noted that, when water and a solvent other than water are used in combination, the ratio between water and the sol vent other than water is not particularly limited as long as the reaction proceeds. When two or more solvents other than water are used, the ratio between/among the two or more solvents other than water is not particularly limited as long as the reaction proceeds. Meanwhile, it is particularly preferred that water is used singly as a solvent or water is used in combination with an organic solvent immiscible with water.

(Reaction Temperature of Process (i))

The reaction temperature of the process (i) is not particularly limited. From the viewpoint of yield, suppression of by-product formation, and economic efficiency, the reaction temperature is, for example, usually in the range of -30°C (minus 30°C) to 50°C, preferably -20°C to 25°C, more preferably -10°C to 10°C.

(Reaction Time of Process (i))

The reaction time of the process (i) is not particularly limited. From the viewpoint of yield, suppression of by-product formation, and economic efficiency, the reaction time is, for example, usually in the range of 0.5 hours to 48 hours, preferably 1 hour to 24 hours, more preferably 1 hour to 12 hours.

(Product of Process (i))

A product of the process (i) is a diazonium salt compound corresponding to the aniline compound of the general formula (1) used as a raw material. The diazonium salt compound is a compound generally well-known to those skilled in the art.

In the present invention, the term "product of the process (i)" includes not only a purified or isolated reaction product of the process (i) but also an unrefined crude product and a mixture containing a reaction product of the process (i). Such a mixture may be, for example, a reaction mixture itself of the process (i) or only a layer, e.g., a water layer, of the reaction mixture containing a reaction product of the process (i). Such a mixture may further be refined, if necessary.

(Process (ii))

Hereinafter, a process (ii) will be described.

The process (ii) is a process in which the product of the process (i) is reacted in the presence of a metal nitrite and a copper compound to manufacture a compound represented by the general formula (1):

[Chemical Formula 7]

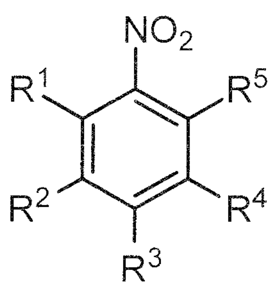

(1)

wherein $R^1$, $R^2$, $R^3$, $R^4$, and $R^5$ are as defined above.

(Metal Nitrite Used in Process (ii))

Examples of the metal nitrite that can be used in the process (ii) include, but are not limited to: an alkali metal nitrite (e.g., lithium nitrite, sodium nitrite, or potassium nitrite); and an alkaline-earth metal nitrite (e.g., magnesium nitrite, calciumnitrite, or barium nitrite). From the viewpoint of price, availability, and reactivity, an alkali metal nitrite is preferred, sodium nitrite or potassium nitrite is more preferred, and sodium nitrite is even more preferred.

The metal nitrite may be in any form as long as the reaction proceeds. As a form of metal nitrite, a solid consisting of solely metal nitrite(s), an aqueous solution of metal nitrite (s) of any concentration, or a solution of metal nitrite(s) solved by a solvent other than water is exemplified. Further, the alkali metal nitrites may be used singly or in combination of two or more species of them at any ratio.

(Amount of Metal Nitrite Used in Process (ii))

The amount of the metal nitrite used is not particularly limited as long as the reaction proceeds. From the viewpoint of yield, suppression of by-product formation, and economic efficiency, the amount of the metal nitrite used is, for example, usually in the range of 1.0 to 10.0 mol, preferably 1.0 to 7.0 mol, more preferably 1.0 to 5.0 mol, even more preferably 2.0 to 4.0 mol per 1 mol of the aniline compound of the general formula (2).

(Copper Compound Used in Process (ii))

In the process (ii), a copper compound is preferably used as a catalyst. As a copper compound that can be used in the process (ii), one generally known as a catalyst for Sandmeyer reaction can be mentioned. Further, a catalyst used in a method proposed by Hantzsch et al. can be mentioned. Examples of the copper compound include copper oxide, a copper(I) salt, a copper(II) salt, and a double salt including copper(I) and copper(II), and copper powder. Examples of the copper oxide include copper (I) oxide and copper(II) oxide. Examples of the copper(I) salt include copper(I) sulfate, copper(I) sulfite, copper (I) carbonate, copper (I) chloride, copper(I) bromide, and copper(I) cyanide. Examples of the copper(II) salt include copper(II) sulfate, copper(II) sulfite, copper(II) carbonate, copper(II) chloride, copper(II) bromide, and copper(II) cyanide. An example of the double salt including copper (I) and copper (II) includes copper(I) copper(II) sulfate (cupro-cupri sulfite) that is a catalyst used in a method proposed by Hantzsch et al. Specific examples of the copper compound that can be used in the process (ii) preferably include, but are not limited to, copper(I) oxide, copper(I) sulfate, copper(I) sulfite, copper(I) copper(II) sulfite (cupro-cupri sulfite), copper(I) carbonate, and copper powder, more preferably include copper(I) oxide, copper(I) sulfate, copper(I) sulfite, copper(I) copper(II) sulfite (cupro-cupri sulfite), and copper powder, and even more preferably include copper(I) oxide and copper(I) copper(II) sulfite (cupro-cupri sulfite).

As described above, the copper compound may be either a simple salt or a double salt. Further, the copper compound may be either an anhydride or a hydrate. The copper compound may be in any form as long as the reaction proceeds. As a form of copper compound, a solid consisting of solely copper compound(s), an aqueous solution of copper compound of any concentration, or a solution of copper compound(s) solved by a solvent other than water is exemplified. Further, the copper compounds may be used singly or in combination of two or more species of them at any ratio.

(Amount of Copper Compound Used in Process (ii))

The amount of the copper compound used is not particularly limited as long as the reaction proceeds. From the viewpoint of yield, suppression of by-product formation, economic efficiency, and safety, the amount of the copper compound used is, for example, usually in the range of 0.01 to 5.0 mol, preferably 0.01 to 1.0 mol, more preferably 0.1 to 0.5 mol per 1 mol of the aniline compound of the general formula (2).

(Use of Base)

In the present invention, a base may be added to the reaction system. For example, a base may be added after the completion of the reaction of the process (i) or before the start of the reaction of the process (ii). In other words, the pH of the reaction system of the process (ii) may be adjusted by adding a base, if necessary. The addition of a base, that is, the adjustment of pH may or may not be performed as long as the reaction of the process (ii) smoothly proceeds. Further, the addition of a base may be performed anytime and anywhere (in any reaction container or the like) as long as the reaction of the process (ii) smoothly proceeds.

(Base)

Examples of the base include, but are not limited to: an alkali metal hydroxide (e.g., lithium hydroxide, sodium hydroxide, or potassium hydroxide); an alkaline-earth metal hydroxide (e.g., magnesium hydroxide, calcium hydroxide, or barium hydroxide); an alkali metal carbonate (e.g., lithium carbonate, sodium carbonate, or potassium carbonate); an alkaline-earth metal carbonate (e.g., magnesium carbonate, calciumcarbonate, orbariumcarbonate); analkalimetalhydrogen carbonate (e.g., lithium hydrogen carbonate, sodium hydrogen carbonate, or potassium hydrogen carbonate) ; an alkaline-earth metal hydrogen carbonate (e.g., magnesium hydrogen carbonate, calcium hydrogen carbonate, or barium hydrogen carbonate); a phosphate (e.g., sodium phosphate, potassium phosphate, or calcium phosphate) ; a hydrogen phosphate (e.g., sodium hydrogen phosphate, potassium hydrogen phosphate, or calcium hydrogen phosphate); an alkali metal carboxylate (e.g., sodium formate, potassium formate, lithium acetate, sodium acetate, orpotassium acetate); an alkaline-earth metal carboxylate (e.g., magnesium acetate or calcium acetate); and ammonia. From the viewpoint of price, handleability, reactivity, and yield, examples of the base preferably include an alkali metal hydroxide, an alkali metal carbonate, and an alkali metal hydrogen carbonate, more preferably include an alkali metal hydrogen carbonate. Specific examples of the base preferably include sodium hydroxide, potassium hydroxide, sodium carbonate, potassium carbonate, sodium hydrogen carbonate, and potassium hydrogen carbonate, more preferably include sodium hydrogen carbonate or potassium hydrogen carbonate, and even more preferably include sodium hydrogen carbonate. The base may be in any form as long as the reaction proceeds. As a form of the base, solid or liquid consisting of solely a base, an aqueous solution of a base of any concentration, or a solution of a base solved by a solvent other than water is exemplified. Further, the bases may be used singly or in combination of two or more species of them at any ratio.

(Amount of Base Used)

The amount of the base used can be determined after consideration by those skilled in the art, if necessary. The amount of the base used is not particularly limited as long as the reaction proceeds. From the viewpoint of yield, suppression of by-product formation, and economic efficiency, the amount of the base used is, for example, usually in the range of 0 (zero) to 5 mol, preferably 0 to 1 mol, more preferably 0 to 0.6 mol per 1 mol of the aniline compound of the general formula (2).

(Solvent Used in Process (ii))

From the viewpoint of allowing the reaction to smoothly proceed, the reaction of the process (ii) is preferably performed in the presence of a solvent. The solvent used in the process (ii) is not particularly limited as long as the reaction of the process (ii) proceeds. Examples of the solvent that can be used in the process (ii) include, but are not limited to: water; alcohols (e.g., methanol, ethanol, 2-propanol, and butanol); ethers (e.g., tetrahydrofuran (THF), 1,4-dioxane, diisopropyl ether, dibutyl ether, cyclopentyl methyl ether (CPME), methyl-tert-butyl ether, 1,2-dimethoxyethane (DME), diglyme, and triglyme); nitriles (e.g., acetonitrile); amides (e.g., N,N-dimethylformamide (DMF), N,N-dimethylacetamide (DMAC), N-methylpyrrolidone (NMP); alkyl ureas (e.g., N,N'-dimethylimidazolidinone (DMI)); sulfoxides (e.g., dimethylsulfoxide (DMSO)); sulfones (e.g., sulfolane); ketones (e. g. , acetone and isobutyl methyl ketone (MIBK)); carboxylates (e. g. , ethyl acetate and butyl acetate); carboxylic acids (e. g. , acetic acid) ; aromatic hydrocarbon derivatives (e.g., benzene, toluene, xylene, chlorobenzene, dichlorobenzene, trichlorobenzene, and nitrobenzene); halogenated aliphatic hydrocarbons (e.g., dichloromethane); and combinations of two or more species of these bases at any ratio.

From the viewpoint of price, handleability, reactivity, and yield, examples of the solvent used in the process (ii) preferably include water alone, a combination of water and an aromatic hydrocarbon derivative, a combination of water and a carboxylate, and a combination of water and a halogenated aliphatic hydrocarbon, more preferably include water alone or a combination of water and an aromatic hydrocarbon derivative, and even more preferably include a combination of water and an aromatic hydrocarbon derivative.

Specific examples of the solvent used in the process (ii) preferably include water alone, a combination of water and toluene, a combination of water and xylene, a combination of water and chlorobenzene, a combination of water and dichlorobenzene, a combination of water and ethyl acetate, a combination of water and butyl acetate, and a combination of water and dichloromethane, more preferably include water alone, a combination of water and toluene, a combination of water and xylene, a combination of water and chlorobenzene, and a combination of water and dichlorobenzene, even more preferably include a combination of water and toluene, a combination of water and xylene, a combination of water and chlorobenzene, and a combination of water and dichlorobenzene, and particularly preferably include a combination of water and toluene.

(Amount of Solvent Used in Process (ii))

The amount of the solvent used in the process (ii) is not particularly limited as long as the reaction system can be sufficiently stirred. From the viewpoint of reactivity, suppression of by-product formation, and economic efficiency, the total amount of water used in the process (ii) is, for example, preferably in the range of 1.2 to 2.2 L (liters), more preferably 1.2 to 1.6 L per 1 mol of the aniline compound of the general formula (2). Here, the total amount of water used in the process (ii) refers to the amount of all water present in the reaction system at the time when the reaction of the process (ii) is finished. Therefore, when the acid or the metal nitrite added is in the form of an aqueous solution, not only the amount of water added in the process (ii) but also the amount of water in the aqueous solution is added. Further, when the reaction mixture of the process (i) is directly used, the amount of water in the reaction mixture is also added. For example, the total amount of water used in the process (ii) includes the amount of water in an aqueous solution of the metal nitrite used in the process (ii) and the amount of water in an aqueous solution of the base used in the process (ii), and further includes the total amount of water used in the process (i) when the reaction mixture of the process (i) is directly used. From the same point of view, the amount of the solvent other than water is, for example, usually in the range of 0 (zero) to 5 L (liters), preferably 0.1 to 1 L, more preferably 0.2 to 0.7 L per 1 mol of the aniline compound of the general formula (2). It is to be noted that when water and a solvent other than water are used in combination, the ratio between water and the solvent other than water is not particularly limited as long as the reaction proceeds. When two or more solvents other than water are used, the ratio between/among the two or more solvents is not particularly limited as long as the reaction proceeds.

(Reaction Temperature of Process (ii))

The reaction temperature of the process (ii) is not particularly limited. From the viewpoint of yield, suppression of by-product formation, and economic efficiency, the reaction temperature is, for example, usually in the range of -30°C (minus 30°C) to 70°C, preferably -20°C to 50°C, more preferably -10°C to 35°C.

(Reaction Time of Process (ii))

The reaction time of the process (ii) is not particularly limited. From the viewpoint of yield, suppression of by-product formation, and economic efficiency, the reaction time is, for example, usually in the range of 0.5 hours to 48 hours, preferably 0.5 hours to 24 hours, more preferably 0.5 hours to 12 hours.

(Product of Process (ii): Nitrobenzene Compound of General Formula (1))

Specific examples of the nitrobenzene compound of the general formula (1) obtained in the process (ii) include, but are not limited to: 2,6-dichloronitrobenzene, 2,6-dibromonitrobenzene, 2,6-difluoronitrobenzene, 2-chloro-6-fluoronitrobenzene, 2-bromo-6-chloronitrobenzene, 2-chloro-6-iodonitrobenzene, methyl 3-chloro-2-nitrobenzoate, ethyl 3-chloro-2-nitrobenzoate, propyl 3-chloro-2-nitrobenzoate, isopropyl 3-chloro-2-nitrobenzoate, butyl 3-chloro-2-nitrobenzoate, isobutyl 3-chloro-2-nitrobenzoate, sec-butyl 3-chloro-2-nitrobenzoate, tert-butyl 3-chloro-2-nitrobenzoate, methyl 3-fluoro-2-nitrobenzoate, ethyl 3-fluoro-2-nitrobenzoate, propyl 3-fluoro-2-nitrobenzoate, isopropyl 3-fluoro-2-nitrobenzoate, butyl 3-fluoro-2-nitrobenzoate, isobutyl 3-fluoro-2-nitrobenzoate, sec-butyl 3-fluoro-2-nitrobenzoate, tert-butyl 3-fluoro-2-nitrobenzoate, methyl 3-bromo-2-nitrobenzoate, ethyl3-bromo-2-nitrobenzoate, propy3-bromo-2-nitrobenzoate, isopropyl 3-bromo-2-nitrobenzoate, methyl 3-iodo-2-nitrobenzoate, and ethyl 3-iodo-2-nitrobenzoate.

(Yield in Process (i) and Process (ii))

The overall yield in the process (i) and the process (ii) in the method according to the present invention is, for example, usually in the range of 70% to 100%, preferably 80% to 100%, more preferably 85% to 100%.

The overall yield in the process (i) and the process (ii) can be calculated as a ratio of the number of moles of the obtained nitrobenzene compound of the general formula (1) to the number of moles of the aniline compound of the general formula (2) used as a raw material. That is, the yield is represented by the following formula:

Yield (%) = 100 × {(number of moles of obtained nitrobenzene compound of general formula (1))/(number of moles of aniline compound of general formula (2) as raw material)}

## Examples

Hereinafter, the producing method according to the present invention will be specifically described with reference to Examples, but is not intended that the present invention is limited thereto.

### Example 1

Production of Methyl 3-Chloro-2-Nitrobenzoate

A 100-mL four-necked flask equipped with a stirrer, a reflux condenser, a thermometer, and a dropping funnel was prepared, and 9.28 g (0.050 mol) of methyl 2-amino-3-chlorobenzoate, 24 mL of water, and 10.95 g (0.105 mol) of 35% hydrochloric acid were added to the flask to obtain a mixture. Then, 9.35 g (0.0515 mol) of a 38% aqueous sodium nitrite solution was dropped into the mixture while the mixture was stirred at -5°C (minus 5°C). Then, the resulting mixture was stirred at -5 to 0°C for 2 hours to obtain a reaction mixture. A 200-mL four-necked flask equipped with a stirrer, a reflux condenser, a thermometer, and a dropping funnel was prepared, and 2.42 g (0.0169 mol) of copper(I) oxide, 25 mL of toluene, 27.24 g (0.150 mol) of a 38% aqueous sodium nitrite solution, and 21 g of water were added to the flask, and then the reaction mixture obtained above was dropped thereinto at 25°C in 2 hours. Then, 25 mL of toluene was added to the resulting reaction mixture, and the resulting mixture was stirred at 30°C for 10 minutes, and then copper was removed by filtration. The resulting filtrate was divided into water and toluene, and then a toluene layer was separated. The obtained toluene layer was washed with 25 mL of water to obtain a toluene solution of methyl 3-chloro-2-nitrobenzoate. The obtained toluene solution was analyzed by an HPLC absolute calibration curve method. As a result, the yield of methyl 3-chloro-2-nitrobenzoate was 88%. At this time, the content of methyl 2,3-dinitrobenzoate as an impurity was 0.6%.

### Example 2

Production of Methyl 3-Chloro-2-Nitrobenzoate

A 100-mL four-necked flask equipped with a stirrer, a reflux condenser, a thermometer, and a dropping funnel was prepared, and 9.28 g (0.050 mol) of methyl 2-amino-3-chlorobenzoate, 24 mL of water, and 10.95 g (0.105 mol) of 35% hydrochloric acid were added to the flask to obtain a mixture. Then, 9.35 g (0.0515 mol) of a 38% aqueous sodium nitrite solution was dropped into the mixture while the mixture was stirred at -5°C (minus 5°C). Then, the resulting mixture was stirred at -5 to 0°C for 2 hours to obtain a reaction mixture. A 200-mL four-necked flask equipped with a stirrer, a reflux condenser, a thermometer, and a dropping funnel was prepared, and 2.42 g (0.0169 mol) of copper(I) oxide, 25 mL of toluene, 27.24 g (0.150 mol) of a 38% aqueous sodium nitrite solution, and 21 g (0.025 mol) of a 10% aqueous sodium hydrogen carbonate solution were added to the flask, and then the reaction mixture obtained above was dropped thereinto at 25°C in 2 hours. Then, 25 mL of toluene was added to the resulting reaction mixture, and the resulting mixture was stirred at 30°C for 10 minutes, and then copper was removed by filtration. The resulting filtrate was divided into water and toluene, and then a toluene layer was separated. The obtained toluene layer was washed with 25 mL of water to obtain a toluene solution of methyl 3-chloro-2-nitrobenzoate. The obtained toluene solution was analyzed by an HPLC absolute calibration curve method. As a result, the yield of methyl 3-chloro-2-nitrobenzoate was 84%. At this time, the content of methyl 2,3-dinitrobenzoate as an impurity was 0.5%.

### Example 3

Production of Methyl 3-Chloro-2-Nitrobenzoate

A 100-mL four-necked flask equipped with a stirrer, a reflux condenser, a thermometer, and a dropping funnel was prepared, and 9.28 g (0.050 mol) of methyl 2-amino-3-chlorobenzoate, 21.6 mL of isobutyl methyl ketone (MIBK), 10 mL of water, and 11.47 g (0.110 mol) of 35% hydrochloric acid were added to the flask to obtain a mixture. Then, 9.81 g (0.054 mol) of a 38% aqueous sodium nitrite solution was dropped into the mixture while the mixture was stirred at -5°C (minus 5°C). Then, the resulting mixture was stirred at -5 to 0°C for 2 hours to obtain a reaction mixture. The mixture was divided into isobutyl methyl ketone and water, and then a water layer was separated. A 200-mL four-necked flask equipped with a stirrer, a reflux condenser, a thermometer, and a dropping funnel was prepared, and 2.42 g (0.0169 mol) of copper(I) oxide, 25 mL of toluene, 27.24g (0.150mol) of a 38% aqueous sodium nitrite solution, and 31 g (1.72 mol) of water were added to the flask, and then the water layer obtained above was dropped thereinto at 0 to 5°C in 2 hours. Then, 25 mL of toluene was added to the resulting reaction mixture, and the resulting mixture was stirred at 30°C for 10 minutes, and then copper was removed by filtration. The resulting filtrate was divided into water and toluene, and

then a toluene layer was separated. The obtained toluene layer was washed with 25 mL of water to obtain a toluene solution of methyl 3-chloro-2-nitrobenzoate. The obtained toluene solution was analyzed by an HPLC absolute calibration curve method. As a result, the yield of methyl 3-chloro-2-nitrobenzoate was 85%. At this time, the content of methyl 2,3-dinitrobenzoate as an impurity was 0.6%.

In Example 1(1) in Patent Document 5, the reaction corresponding to the process (ii) in the method according to the present invention is performed under conditions where the total amount of water used is 2.4 L (liters) per 1 mol of the aniline compound of the general formula (2). In Non Patent Document 1, the reaction corresponding to the process (ii) in the method according to the present invention is performed under conditions where the total amount of water used is 2.5 L (liters) per 1 mol of the aniline compound of the general formula (2). In Patent Document 5 and Non Patent Document 1, the amount of water used is large, and therefore economic efficiency is low.

In Comparative Example 1 that will be described later, the reaction of the process (ii) was performed under conditions where the total amount of water used was about 1.0 L per 1 mol of the aniline compound of the general formula (2). As a result, production efficiency was improved, but methyl 2,3-dinitrobenzoate was formed as a by-product in an amount as large as 3.3%. Methyl 2,3-dinitrobenzoate is a dangerous dinitro compound. Therefore, the method of Comparative Example 1 is not industrially favorable.

On the other hand, in Example 1 of the present invention, the reaction of the process (ii) was performed under conditions where the total amount of water used was about 1.5 L per 1 mol of the aniline compound of the general formula (2). As a result, the amount of methyl 2,3-dinitrobenzoate formed as a by-product was as small as 0.6%. That is, in Example 1, the amount of a dangerous dinitro compound could be significantly reduced by increasing the total amount of water used in the process (ii) as compared to that in Comparative Example 1. In addition, the total amount of water used in Example 1 of the present invention is much smaller than that in Patent Document 5 and Non patent Document 1. Therefore, the method according to the present invention showed a significant improvement in economic efficiency as compared to conventional art. According to the present invention, multiple advantages were achieved by appropriately adjusting the amount of water.

In Example 2 of the present invention, the total amount of water used in the process (ii) was adjusted to about 1.5 L per 1 mol of the aniline compound of the general formula (2) as in the case of Example 1. In addition, a base was added. An improvement of yield, which was achieved by adding a base in Example 5 that will be described later, was not observed in Example 2, however, the yield in Example 2 was sufficient and acceptable. Further, in spite of the fact that a base was added, the amount of dangerous methyl 2,3-dinitrobenzoate was as small as 0.5% because the total amount of water used was appropriately adjusted.

Also in Example 3 of the present invention, the total amount of water used in the process (ii) was adjusted to about 1.4 L per 1 mol of the aniline compound of the general formula (2). The amount of dangerous methyl 2,3-dinitrobenzoate was small because the total amount of water used was appropriately adjusted. In addition, the total amount of water used in Example 3 of the present invention is much smaller than that in Patent Document 5 and Non Patent Document 1. Therefore, economic efficiency is improved as compared to conventional art.

Example 4

Production of 2,6-dichloronitrobenzene

A 1000-mL four-necked flask equipped with a stirrer, a reflux condenser, a thermometer, and a dropping funnel was prepared, and 100.5 g (0.62 mol) of 2,6-dichloroaniline, 296.1 mL of water, and 160.2 g (1.54 mol) of 35% hydrochloric acid were added to the flask to obtain a mixture. The mixture was heated to 60°C and melted. The mixture was cooled to -5°C (minus 5°C) while stirred, and then 144.1 g (0.79 mol) of a 38% aqueous sodium nitrite solution was dropped thereinto at -5 to 0°C to obtain a reaction mixture. A 2000-mL four-necked flask equipped with a stirrer, a reflux condenser, a thermometer, and a dropping funnel was prepared, and 30.0 g (0.21 mol) of copper (I) oxide, 216.7 mL of toluene, and 675.5 g (3.72 mol) of a 38% aqueous sodium nitrite solution were added to the flask, and the reaction mixture obtained above was dropped thereinto at 0 to 5°C in 2 hours. The resulting mixture was stirred at 0 to 5°C for 30 minutes, and then a filter aid was added and copper was removed by filtration. The resulting filtrate was divided into toluene and water, and then a toluene layer was separated. The obtained toluene layer was washed with 208.3 mL (0.25 mol) of a saturated aqueous sodium hydrogen carbonate solution to obtain a toluene solution of 2,6-dichloronitrobenzene. The obtained toluene solution was analyzed by a GC internal reference method. As a result, the yield of 2,6-dichloronitrobenzene was 73%.

Example 5

Production of 2,6-dichloronitrobenzene

A 2000-mL four-necked flask equipped with a stirrer, a reflux condenser, a thermometer, and a dropping funnel was prepared, and 162.0 g (1.00 mol) of 2, 6-dichloroaniline, 396.4 mL of water, and 239.6 g (2.30 mol) of 35% hydrochloric acid were added to the flask to obtain a mixture. The mixture was heated to 60°C and melted. The mixture was cooled to -5°C (minus 5°C) while stirred, and then 199.7 g (1.10 mol) of a 38% aqueous sodium nitrite solution was dropped there into at -5 to 0°C. Then, 174.7 mL of toluene was added, and 420 mL of a saturated aqueous sodium hydrogen carbonate solution was dropped at -5 to 0°C to adjust pH to 3. The resulting mixture was divided into toluene and water, and then a water layer was separated. A 3000-mL four-necked flask equipped with a stirrer, a reflux condenser, a thermometer, and a dropping funnel was prepared, and 48.4 g (0.34 mol) of copper(I) oxide, 349.5 mL of toluene, and 544.7 g (3.00 mol) of a 38% aqueous sodium nitrite solution were added to the flask, and the water layer obtained above was dropped thereinto at 0 to 5°C in 2 hours. The resulting mixture was stirred at 0 to 5°C for 30 minutes, and then copper was removed by filtration. The resulting filtrate was divided into toluene and water, and then a toluene layer was separated. The obtained toluene layer was washed with 336.0 mL (0.40 mol) of a saturated aqueous sodium hydrogen carbonate solution to obtain a toluene solution of 2,6-dichloronitrobenzene. The obtained toluene solution was analyzed by a GC internal reference method. As a result, the yield of 2,6-dichloronitrobenzene was 85%.

In Example 4, the reaction of the process (ii) was performed under conditions where the total amount of water used was about 1.5 L per 1 mol of the aniline compound of the general formula (2). The total amount of water used in Example 4 of the present invention was much smaller than that in Patent Document 5 and Non Patent Document 1. In addition, the yield of 2,6-dichloronitrobenzene was as sufficient as 73%. Therefore, the method according to the present invention showed a significant improvement in economic efficiency as compared to conventional art.

In Example 5, the total amount of water used in the process (ii) was adjusted to about 1.4 L per 1 mol of the aniline compound of the general formula (2), which was almost the same as in Example 4. In addition, a base was added. As a result, the yield in Example 5 was increased to 85% by as much as 12%, whereas the yield in Example 4 was 73%. This value is industrially and commercially significant.

In Example 1 (1) of Patent Document 5 and Non Patent Document 1, 6 mol of sodium nitrite is used per 1 mol of the aniline compound of the general formula (2) in the process corresponding to the process (ii) of the method according to the present invention. On the other hand, the amount of sodium nitrite used in the process (ii) in Examples 1, 2, 3, and 5 of the present invention is 3 mol per 1 mol of the aniline compound of the general formula (2). That is, the amount of sodium nitrite used in Examples 1, 2, 3, and 5 of the present invention is much smaller than that in Patent Document 5 and Non Patent Document 1. Also from such a viewpoint, the present invention provides a method having superiority over conventional art.

Comparative Example 1

Production of methyl 3-chloro-2-nitrobenzoate

A 100-mL four-necked flask equipped with a stirrer, a reflux condenser, a thermometer, and a dropping funnel was prepared, and 9.28 g (0.050 mol) of methyl 2-amino-3-chlorobenzoate, 24 mL of water, and 10.95 g (0.105 mol) of 35% hydrochloric acid were added to the flask to obtain a mixture. Then, 9.35 g (0.0515 mol) of a 38% aqueous sodium nitrite solution was dropped into the mixture while the mixture was stirred at -5°C (minus 5°C). Then, the resulting mixture was stirred at -5 to 0°C for 2 hours to obtain a reaction mixture. A 200-mL four-necked flask equipped with a stirrer, a reflux condenser, a thermometer, and a dropping funnel was prepared, and 2.42 g (0.0169 mol) of copper(I) oxide, 25 mL of toluene, and 27.24 g (0.150 mol) of a 38% aqueous sodium nitrite solution were added to the flask, and the reaction mixture obtained above was dropped thereinto at 25°C in 2 hours. Then, 25 mL of toluene was added to the resulting reaction mixture, and the resulting mixture was stirred at 30°C for 10 minutes, and then copper was removed by filtration. The resulting filtrate was divided into toluene and water, and then a toluene layer was separated. The obtained toluene layer was washed with 25 mL of water to obtain a toluene solution of methyl 3-chloro-2-nitrobenzoate. The obtained toluene solution was analyzed by an HPLC absolute calibration curve method. As a result, the yield of methyl 3-chloro-2-nitrobenzoate was 85%. At this time, the content of methyl 2,3-dinitrobenzoate as an impurity was 3.3%.

(High-Performance Liquid Chromatography (HPLC) Analysis Method)

The following documents can be referred to for the HPLC analysis method, as desired.

(a) : "Shin-Jikkenkagaku Koza 9, Bunseki Kagaku II (A New Course in Experimental Chemistry 9, Analytical Chemistry II)", pp. 86 to 112 (1977), edited by The Chemical Society of Japan, published by Iizumi Shingo, Maruzen Company, Limited (forexample, it is possible to refer to pages 93 to 96 of this document with respect to a filler-mobile phase combination to be usable for a column)

(b): "Jikkenkagaku Koza 20-1, Bunseki Kagaku (A Course in Experimental Chemistry 20-1, Analytical Chemistry)", 5th edition, pp. 130 to 151 (2007), edited by The Chemical Society of Japan, published by Murata Seishiro, Maruzen Company, Limited (for example, it is possible to refer to pages 135 to 137 of this document with respect to the specific method and conditions for reverse phase chromatography analysis)

(Gas Chromatography (GC) Analysis Method)

The following documents can be referred to for the GC analysis method, as desired.

(a) : "Shin-Jikkenkagaku Koza 9, Bunseki Kagaku II (A New Course in Experimental Chemistry Course 9, Analytical Chemistry II)", pp. 60 to 86 (1977), edited by The Chemical Society of Japan, published by Iizumi Shingo, Maruzen Company, Limited (for example, it is possible to refer to page 66 of this document with respect to liquids for a stationary phase to be usable for a column)

(b): "Jikkenkagaku Koza 20-1, Bunseki Kagaku (A Course in Experimental Chemistry 20-1, Analytical Chemistry)", 5th edition, pp. 121 to 129 (2007), edited by The Chemical Society of Japan, published by Murata Seishiro, Maruzen Company, Limited (for example, it is possible to refer to pages 124 to 125 of this document with respect to the specific usage of hollow capillary separation columns)

(Method for Measuring pH)

The pH was measured by a glass electrode-type hydrogen-ion concentration indicator. As the glass electrode-type hydrogen-ion concentration indicator, for example, Model HM-20P manufactured by DKK-TOA CORPORATION can be used.

Industrial Applicability

According to the present invention, it is possible to provide a novel method for producing a nitrobenzene compound of the general formula (1).

According to the present invention, a nitrobenzene compound of the general formula (1) can be produced while the amount of water used is reduced as compared to conventional art. Therefore, the method according to the present invention can improve economic efficiency as compared to conventional art.

Further, according to the present invention, the amount of a dangerous dinitro compound formed as a by-product can be reduced by appropriately adjusting the amount of water used. Therefore, the method according to the present invention provides improved safety. Further, the present invention can produce a high-purity nitrobenzene compound of the general formula (1).

That is, according to the present invention, it is possible to provide a method for producing a nitrobenzene compound of the general formula (1), which includes appropriately adjusting the amount of water used and is therefore economically favorable and suitable for industrialization.

Further, according to the present invention, it is possible to produce a nitrobenzene compound of the general formula (1) without using hydrogen peroxide.

Further, the present invention provides a method for producing a nitrobenzene compound of the general formula (1) using a significantly-reduced amount of a metal nitrite as compared to conventional art.

Further, another aspect of the present invention provides a method including adding a base. In other words, in another aspect of the present invention, the pH of a reaction system can be adjusted by adding a base. The present invention can achieve a sufficient and acceptable yield without using a base. However, there is a case where a higher yield can be achieved by adding a base, as desired.

The method according to the present invention achieves a high yield. In addition, the method according to the present invention can be implemented by simple operation under mild conditions without using a specialized reaction apparatus. Therefore, the method according to the present invention can be efficiently implemented on an industrial scale.

Further, the method according to the present invention is considered to produce a little amount of waste and to have no problem with the properties of the waste.

Therefore, the method according to the present invention is economical and friendly to the environment and has a great deal of potential in industry.

## Claims

1. A method for producing a nitrobenzene compound represented by the general formula (1):

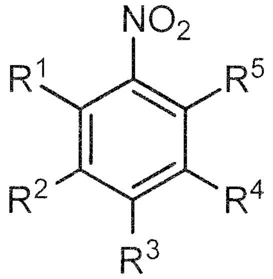

(1)

wherein, in the general formula (1), $R^1$ represents a halogen atom, $R^2$, $R^3$, and $R^4$ are the same or different from one another and each represent a hydrogen atom, a halogen atom, or an alkyl group, and $R^5$ represents a halogen atom or an alkoxycarbonyl group,
the method comprising the processes of:

(i) reacting an aniline compound represented by the general formula (2) :

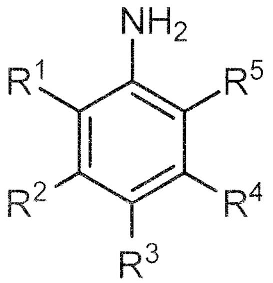

(2)

wherein, in the general formula (2), $R^1$, $R^2$, $R^3$, $R^4$, and $R^5$ are as defined above,
in the presence of a metal nitrite and an acid; and
(ii) reacting a product of the process (i) in the presence of a metal nitrite and a copper compound, wherein a total amount of water used in the process (ii) is 1.2 to 2.2 L per 1 mol of the compound of the general formula (2).

2. The method according to claim 1, wherein the total amount of water used in the process (ii) is 1.2 to 1.6 L per 1 mol of the compound of the general formula (2).

3. The method according to claim 1 or 2, wherein $R^2$, $R^3$, and $R^4$ are hydrogen atoms.

4. The method according to any one of claims 1 to 3, wherein $R^1$ is a halogen atom and $R^5$ is a $C_1$ to $C_4$ alkoxycarbonyl group.

5. The method according to any one of claims 1 to 3, wherein $R^1$ is a chlorine atom and $R^5$ is a $C_1$ to $C_4$ alkoxycarbonyl group.

6. The method according to any one of claims 1 to 3, wherein $R^1$ is a chlorine atom and $R^5$ is methoxycarbonyl group.

7. The method according to any one of claims 1 to 3, wherein $R^1$ is a halogen atom and $R^5$ is a halogen atom.

8. The method according to any one of claims 1 to 3, wherein $R^1$ is a chlorine atom and $R^5$ is a chlorine atom.

## Patentansprüche

1. Verfahren zur Herstellung einer Nitrobenzolverbindung der allgemeinen Formel (1):

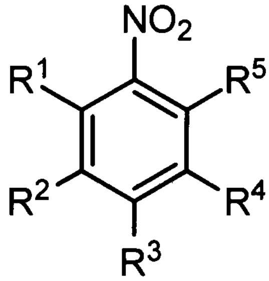

( 1 )

wobei in der allgemeinen Formel (1) gilt: $R^1$ steht für ein Halogenatom, $R^2$, $R^3$ und $R^4$ sind gleich oder verschieden voneinander und stehen jeweils für ein Wasserstoffatom, ein Halogenatom oder eine Alkylgruppe, und $R^5$ steht für ein Halogenatom oder eine Alkoxycarbonylgruppe, wobei das Verfahren folgende Schritte umfasst:

(i) das Umsetzen einer Anilinverbindung der allgemeinen Formel (2):

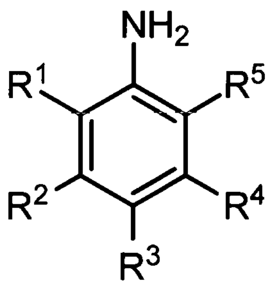

( 2 )

wobei in der allgemeinen Formel (2) gilt: $R^1$, $R^2$, $R^3$, $R^4$ und $R^5$ sind wie oben definiert,
in Gegenwart eines Metallnitrits und einer Säure; und
(ii) das Umsetzen des Produkts des Schritts (i) in Gegenwart eines Metallnitrits und einer Kupferverbindung, wobei die Gesamtmenge des in dem Schritt (ii) verwendeten Wassers 1,2 bis 2,2 l pro 1 mol der Verbindung der allgemeinen Formel (2) beträgt.

2. Verfahren nach Anspruch 1, wobei die Gesamtmenge des in dem Schritt (ii) verwendeten Wassers 1,2 bis 1,6 l pro 1 mol der Verbindung der allgemeinen Formel (2) beträgt.

3. Verfahren nach Anspruch 1 oder 2, wobei $R^2$, $R^3$ und $R^4$ Wasserstoffatome sind.

4. Verfahren nach einem der Ansprüche 1 bis 3, wobei $R^1$ ein Halogenatom ist und $R^5$ eine $C_1$-$C_4$-Alkoxycarbonylgruppe ist.

5. Verfahren nach einem der Ansprüche 1 bis 3, wobei $R^1$ ein Chloratom ist und $R^5$ eine $C_1$-$C_4$-Alkoxycarbonylgruppe ist.

6. Verfahren nach einem der Ansprüche 1 bis 3, wobei $R^1$ ein Chloratom ist und $R^5$ eine Methoxycarbonylgruppe ist.

7. Verfahren nach einem der Ansprüche 1 bis 3, wobei $R^1$ ein Halogenatom ist und $R^5$ ein Halogenatom ist.

8. Verfahren nach einem der Ansprüche 1 bis 3, wobei $R^1$ ein Chloratom ist und $R^5$ ein Chloratom ist.

## Revendications

1. Procédé de production d'un composé de nitrobenzène représenté par la formule générale (I) :

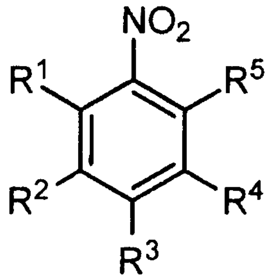

( 1 )

dans lequel, dans la formule générale (I), $R^1$ représente un atome d'halogène, $R^2$, $R^3$ et $R^4$ sont identiques ou différents les uns des autres et chacun représente un atome d'hydrogène, un atome d'halogène, ou un groupe alkyle, et $R^5$ représente un atome d'halogène ou un groupe alcoxycarbonyle,
le procédé comprenant les processus de :

(i) mise en réaction d'un composé d'aniline représenté par la formule générale (2) :

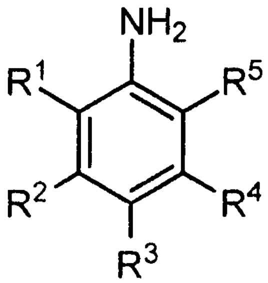

( 2 )

dans lequel, dans la formule générale (2), $R^1$, $R^2$, $R^3$, $R^4$ et $R^5$ sont tels que définis ci-dessus,
en présence d'un nitrite de métal et d'un acide ; et
(ii) mise en réaction d'un produit du procédé (i) en présence d'un nitrile de métal et d'un composé de cuivre, dans lequel une quantité totale d'eau utilisée dans le processus (ii) est de 1,2 à 2,2 L pour 1 mole du composé de formule générale (2).

**2.** Procédé selon la revendication 1, dans lequel la quantité totale d'eau utilisée dans le processus (ii) est de 1,2 à 1,6 L pour 1 mole du composé de formule générale (2).

**3.** Procédé selon la revendication 1 ou 2, dans lequel $R^2$, $R^3$ et $R^4$ sont des atomes d' hydrogène.

**4.** Procédé selon l'une quelconque des revendications 1 à 3, dans lequel $R^1$ est un atome d'halogène et $R^5$ est un groupe alcoxycarbonyle en $C_1$ à $C_4$.

**5.** Procédé selon l'une quelconque des revendications 1 à 3, dans lequel $R^1$ est un atome de chlore et $R^5$ est un groupe alcoxycarbonyle en $C_1$ à $C_4$.

**6.** Procédé selon l'une quelconque des revendications 1 à 3, dans lequel $R^1$ est un atome de chlore et $R^5$ est un groupe méthoxycarbonyle.

**7.** Procédé selon l'une quelconque des revendications 1 à 3, dans lequel $R^1$ est un atome d'halogène et $R^5$ est un atome d'halogène.

**8.** Procédé selon l'une quelconque des revendications 1 à 3, dans lequel $R^1$ est un atome de chlore et $R^5$ est un atome de chlore.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005533756 A
- JP 2008537953 A
- WO 2005081960 A2
- US 5084086 A
- JP 2606291 B
- WO 2013005425 A1


### Non-patent literature cited in the description


- *Transactions of Tianj in University,* 2002, vol. 8 (1), 40-41
- Shin-Jikkenkagaku Koza 9, Bunseki Kagaku II (A New Course in Experimental Chemistry 9, Analytical Chemistry II). Iizumi Shingo, Maruzen Company, Limited, 1977, 86-112
- Jikkenkagaku Koza 20-1, Bunseki Kagaku (A Course in Experimental Chemistry 20-1, Analytical Chemistry). Murata Seishiro, Maruzen Company, Limited, 2007, 130-151
- Shin-Jikkenkagaku Koza 9, Bunseki Kagaku II (A New Course in Experimental Chemistry Course 9, Analytical Chemistry II. Iizumi Shingo, Maruzen Company, Limited, 1977, 60-86
- Jikkenkagaku Koza 20-1, Bunseki Kagaku (A Course in Experimental Chemistry 20-1, Analytical Chemistry. Murata Seishiro, Maruzen Company, Limited, 2007, 121-129